# EUROPEAN PATENT SPECIFICATION

(11) **EP 4 590 389 B1**
(45) Date of publication and mention of the grant of the patent: **28.10.2026**
(21) Application number: 23840789.4
(22) Date of filing: 21.12.2023
(51) Int. Cl.: A61N 1/36, A61N 1/04, A61N 1/40

(54) **MULTI-CHANNEL DEVICE FOR DIRECTIONAL SHAPING OF ELECTRIC FIELDS E.G., TUMOR TREATING FIELDS (TTFIELDS)**
MEHRKANALIGE VORRICHTUNG ZUR GERICHTETEN FORMUNG ELEKTRISCHER FELDER, Z.B. TUMORBEHANDLUNGSFELDER (TTFIELDS)
DISPOSITIF À CANAUX MULTIPLES POUR LA MISE EN FORME DIRECTIONNELLE DE CHAMPS ÉLECTRIQUES, PAR EXEMPLE, DES CHAMPS DE TRAITEMENT DE TUMEUR (TTFIELDS)

(30) Priority: 27.12.2022 US 202263435370 P
(43) Date of publication of application: 30.07.2025
(73) Proprietor: Novocure GmbH, 6340 Baar (CH)
(72) Inventor: DESLAURIERS, Richard, Woodbury, Connecticut 06798 (US)
(74) Representative: Carpmaels & Ransford LLP
(86) International application number: PCT/IB2023/063131
(87) International publication number: WO 2024/141898

(56) References cited:
- CN-A- 113 082 519
- CN-A- 115 006 718
- KR-A- 20220 055 065
- US-A1- 2010 324 547
- US-A1- 2018 001 078
- US-A1- 2021 370 063

## Description

The present application relates to an apparatus for applying an electric field to a target region of a subject.

### BACKGROUND

Tumor Treating Fields (TTFields) therapy is a proven approach for treating tumors using alternating electric fields at frequencies between 50 kHz and 1 MHz (e.g., 150-200 kHz). See, for example, US Patent No. 7,565,205. Alternating electric fields can also be used to treat medical conditions other than tumors. For example, as described in U.S. Patent No. 10,967,167, alternating electric fields can be used to increase the permeability of the blood brain barrier so that, e.g., chemotherapy drugs can reach the brain.

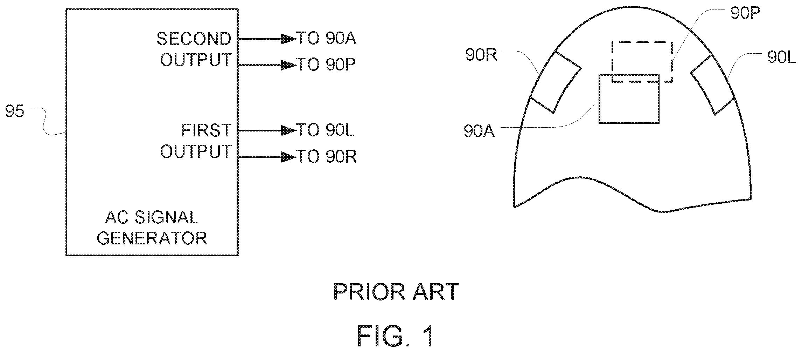

FIG. 1 depicts the prior art Optune^{®} system, which delivers TTFields to patients via four transducer arrays 90 that are placed on the patient's skin near the tumor. The transducer arrays 90 are arranged in two pairs, with one pair of transducer arrays 90L, 90R positioned to the left and right of the tumor, and the other pair of transducer arrays 90A, 90P positioned anterior and posterior to the tumor. Each transducer array is connected via a multiwire cable to an AC signal generator 95. The AC signal generator (a) sends an AC current through the anterior/posterior (A/P) pair of transducer arrays for 1 second, which induces an electric field with a first direction through the tumor; then (b) sends an AC current through the left/right (L/R) pair of arrays for 1 second, which induces an electric field with a second direction through the tumor; then repeats steps (a) and (b) for the duration of the treatment. Each transducer array includes a plurality (e.g., between 9 and 30) of electrode elements. And all of the electrode elements on any given transducer array are wired together (e.g., in series or in parallel).

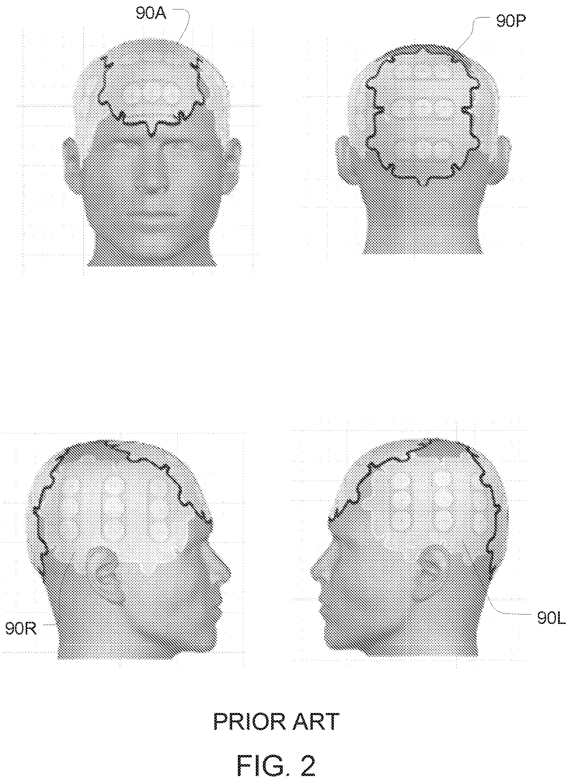

FIG. 2 depicts a more detailed view of a set of four transducer arrays 90 in which the individual electrode elements that make up each of the transducer arrays are visible. In this example, each of the transducer arrays 90 includes nine round electrode elements that are supported by a self-adhesive substrate. In FIG. 2, each of the four transducer arrays 90 is positioned at a particular place on a patient's head.

Increasing the strength of alternating electric fields in the relevant target region (e.g., in a tumor such as a glioblastoma) will typically increase the efficacy of treatment. And it is often possible to increase the strength of the fields in the target region by shifting the positions of the transducer arrays 90 away from the exact positions depicted in FIG. 2. More specifically, shifting the positions of the transducer arrays 90 on the relevant body part (e.g., by a few centimeters in the up, down, right, left, front, and/or back directions) can change the paths of the alternating electric fields that travel through the body part. And changing the paths of the electric fields can increase in field strength in the target region, which can in turn increase the efficacy of treatment.

But it turns out that the degree of control over the paths of the electric fields that is achievable by shifting the positions of the transducer arrays is limited. And this in turn limits how much the field strength can be increased.

CN-A-113082519 discloses a device for destroying diseased cells or inhibiting division of diseased cells, which comprises a plurality of groups of electrode patch pairs which surround a target tissue, a voltage generator which is connected with the electrode patch pairs and outputs voltage signals to the electrode patch pairs, and a controller which is connected with the voltage generator and the electrode patch pairs and controls the direction of an electric field between the electrode patch pairs so as to form a space electric field at least surrounding the target tissue.

KR-A-2022/0055065 discloses a device for inducing apoptosis of abnormally dividing cells, which includes a first array electrode, a second array electrode and a power source for supplying electric power to drive the first and second array electrodes.

US-A-2021/0370063 discloses neurostimulation devices and methods which provide a plurality of electrodes placed around the head of a patient, and a controller which controls current between sets of opposing electrodes and through the brain to selectively stimulate a region of interest of the brain.

### SUMMARY OF THE INVENTION

One aspect of the invention is directed to an apparatus for applying an electric field to a target region of a subject according to claim 1.

Embodiments of the invention are disclosed in the dependent claims.

### BRIEF DESCRIPTION OF THE DRAWINGS

FIG. 1 is a block diagram of the prior art Optune^{®} system for delivering TTFields.
FIG. 2 is a more detailed view of the transducer arrays of FIG. 1.
FIG. 3 depicts a set of four transducer arrays positioned on a patient's skin near a tumor.
FIG. 4 is a block diagram of a system for applying electrical signals to the electrode elements in the transducer arrays depicted in FIG. 3.
FIG. 5 depicts controlling the phases of signals that are generated by a set of signal generators to produce a specific set of output signals.

Various embodiments are described in detail below with reference to the accompanying drawings, wherein like reference numerals represent like elements.

### DESCRIPTION OF THE PREFERRED EMBODIMENTS

This application discloses an alternative approach for shifting the paths of the alternating electric fields that travel through the relevant body part. And this approach can, in many situations, increase in field strength in the target region beyond the level that can be attained using the prior art approach of shifting the positions of the transducer arrays.

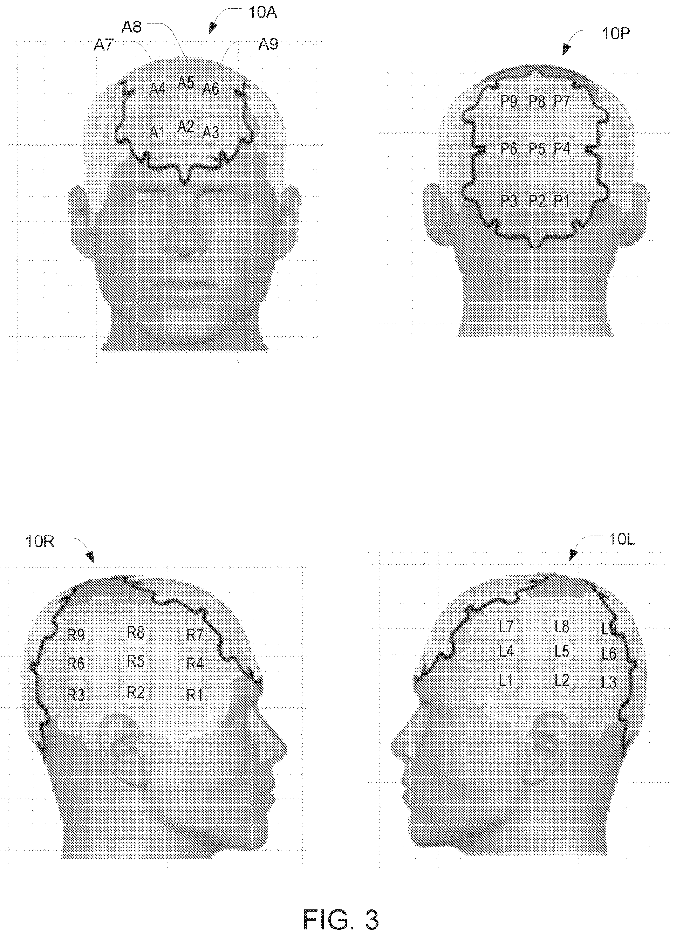

More specifically, instead of using a single signal generator to apply a signal between all of the electrode elements on one side of the target region and all of the electrode elements on the opposite side of the target region (as in the prior art), the embodiments described below in connection with FIGS. 3-5 use at least eight electrically isolated signal generators to apply electrical signals between respective electrode elements on one side of the target region and respective electrode elements on the opposite side of the target region. This replaces the prior art's single wide-cross-section electric field with eight independently controllable electric fields, each of which has a relatively narrow cross-section. Using these relatively narrow cross-section fields, either alone or in combination, can improve aiming of the electric field, which can in turn increase the field strength in the target region. And optionally, activation of these relatively narrow cross-section fields can be shifted in time (e.g., phase-shifted) to achieve steering of the overall electric field.

FIG. 3 depicts a set of four transducer arrays 10 that are placed on the patient's skin near the tumor. The transducer arrays 10 are arranged in two pairs, with one pair of transducer arrays 10L, 10R positioned to the left and right of the tumor, and the other pair of transducer arrays 10A, 10P positioned anterior and posterior to the tumor. Each of the transducer arrays 10 is, in most respects, similar to the prior art Optune^{®} transducer arrays 90 described above. But unlike the Optune^{®} transducer arrays, all of the electrode elements on any given transducer array 10 are not wired together in series or in parallel. Instead, each of the electrode elements A1-A9 on the anterior transducer array 10A is provided with its own individual wire so it can be driven independently. And similarly, each of the electrode elements P1-P9, L1-L9, and R1-R9 on the posterior, left, and right arrays 10P, 10L, 10R is provided with its own respective individual wire so it can be driven independently.

Providing an individual wire for each electrode element on each of the transducer arrays 10 enables an individualized signal to be applied it to each of the electrode elements on each transducer array independently, without affecting the other electrode elements on the transducer array. And the benefits provided by this arrangement are described below.

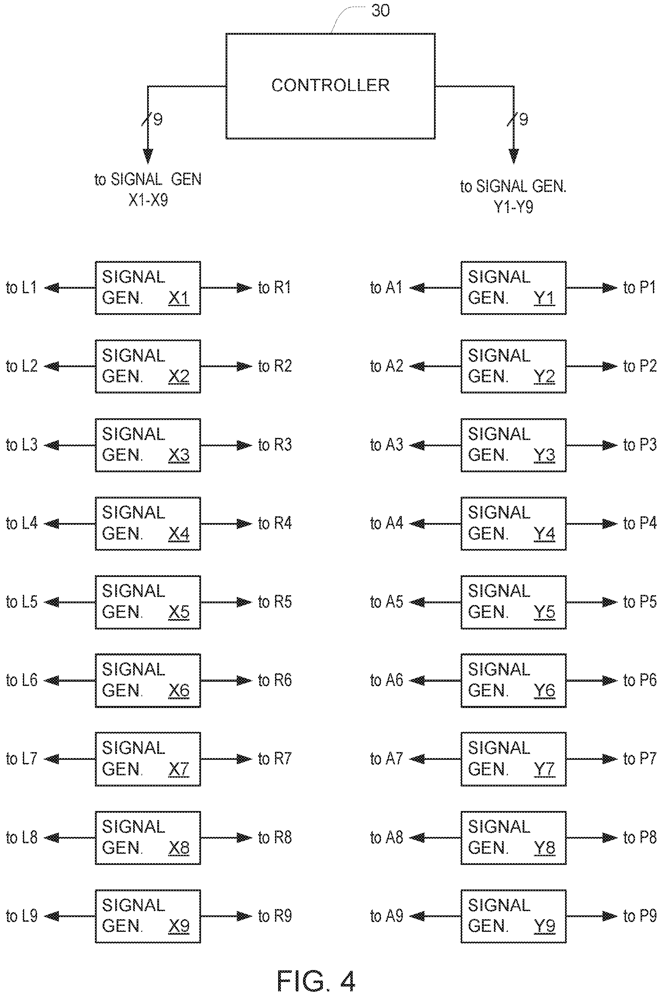

FIG. 4 is a block diagram of a system for applying electrical signals to the electrode elements A1-A9, P1-P9, L1-L9, and R1-R9 depicted in FIG. 3. The system in the illustrated embodiment includes nine first signal generators X1-X9. And each of these first signal generators X1-X9 is wired up to apply a signal between a respective single one of the electrode elements L1-L9 on the left array 10L and a respective single one of the electrode elements R1-R9 on the right array 10R. More specifically, signal generator Xi is wired to apply a signal between electrode element Li and electrode element Ri, where i is an integer between 1 and 9. In some embodiments, the output of any given one of the first signal generators Xi can be either positive (i.e., where the respective L terminal is the anode and the respective R terminal is the cathode), negative (i.e. where the respective L terminal is the cathode and the respective R terminal is the anode), or off (i.e. where no pulse is generated).

Each of the first signal generators X1-X9 is electrically isolated from all the other signal generators. As a result, the signal that signal generator X1 applies between electrode element L1 and electrode element R1 will not affect the signals that are applied to any of the other electrode elements L2-L9, R2-R9, A1-A9, or P1-P9. A similar situation exists for all the other first signal generators X2-X9, so that the signals that those signal generators send to their respective electrode elements will not affect the signals that are applied to any of the other electrode elements.

Each of the first signal generators X1-X9 has a respective first control input, and each of the first signal generators X1-X9 is configured to, in response to a respective first control signal that arrives at the respective first control input, apply an electrical signal between a respective one of the electrode elements L1-L9 and a respective one of the electrode elements R1-R9. A controller 30 is programmed to generate each of these first control signals.

Each of the first signal generators X1-X9 can be configured to, (a) in response to a respective first control signal that arrives at the respective first control input, apply a positive electrical signal between the respective one of the electrode elements L1-L9 and the respective one of the electrode elements R1-R9, and (b) in response to a respective second control signal that arrives at the respective second control input, apply a negative electrical signal between the respective one of the electrode elements L1-L9 and the respective one of the electrode elements R1-R9. Note that the use of the identifiers (a) and (b) does not imply that (a) must precede (b) in time.

When the first signal generators are implemented as described in the previous paragraph, the controller 30 can be programmed to, for each of the first signal generators X1-X9, apply the respective first control signal and the respective second control signal at respective times in an alternating sequence so that the respective first signal generator will generate an output that alternates between a positive electrical signal and a negative electrical signal. Optionally, in these embodiments, the controller 30 can be further programmed to interpose a break in time between each first control signal and each second control signal.

The system in the illustrated embodiment also includes nine second signal generators Y1-Y9. And each of these second signal generators Y1-Y9 is wired up to apply a signal between a respective single one of the electrode elements A1-A9 on the anterior array 10A and a respective single one of the electrode elements P1-P9 on the posterior array 10P. More specifically, signal generator Yj is wired to apply a signal between electrode element Aj and electrode element Pj, where j is an integer between 1 and 9. In some embodiments, the output of any given one of the second signal generators Yj can be either positive (i.e., where the respective A terminal is the anode and the respective P terminal is the cathode), negative (i.e. where the respective A terminal is the cathode and the respective P terminal is the anode), or off (i.e. where no pulse is generated).

Each of the second signal generators Y1-Y9 is electrically isolated from all the other signal generators. As a result, the signal that signal generator Y1 applies between electrode element A1 and electrode element P1 will not affect the signals that are applied to any of the other electrode elements A2-A9, P2-P9, L1-L9, or R1-R9. A similar situation exists for all the other second signal generators Y2-Y9, so that the signals that those signal generators send to their respective electrode elements will not affect the signals that are applied to any of the other electrode elements.

Each of the second signal generators Y1-Y9 has a respective second control input, and each of the second signal generators Y1-Y9 is configured to, in response to a respective second control signal that arrives at the respective second control input, apply an electrical signal between a respective one of the electrode elements A1-A9 and a respective one of the electrode elements P1-P9. The controller 30 is further programmed to generate each of these second control signals. Operation of the second signal generators Y1-Y9 is similar to the operation of the first signal generators X1-X9 described above.

In the FIG. 1 prior art system, where all of the electrode elements on the left transducer array 90L are wired together in series or in parallel, and all of the electrode elements on the right transducer array 90R are wired together in series or parallel, a single electric field travels from all the electrode elements on the left transducer array 90L to all of the electrode elements on the right transducer array 90R. And a single electric field travels from all the electrode elements on the anterior transducer array 90A to all of the electrode elements on the posterior transducer array 90P. The resulting electric fields will therefore have comparatively large cross-sections, and can therefore be analogized to floodlights.

In contrast, in the FIG. 3/4 embodiment, one electric field travels from electrode element L1 to electrode element R1 when signal generator X1 is activated, a second electric field travels from electrode element L2 to electrode element R2 when signal generator X2 is activated, a third electric field travels from electrode element L3 to electrode element R3 when signal generator X3 is activated, etc. As a result, nine distinct electric fields can travel from respective electrode elements L1-L9 to respective electrode elements R1-R9, depending on which ones of the signal generators X1-X9 are activated. When any given single one of the signal generators X1-X9 is activated, the resulting electric field will have a comparatively small cross-section and can therefore be analogized to a spotlight.

Notably, more than one of the signal generators X1-X9 can be activated simultaneously (e.g., any 2 at a time, any 3 at a time, etc., up to all 9 at a time). When all nine of the signal generators X1-X9 are activated simultaneously, the resulting electric field will have a cross-section that is similar to the electric field that was generated using the prior art transducer arrays 90L/90R. The latter situation can be analogized to the situation where the light from nine individual spotlights collectively illuminate the same area as a floodlight.

Similarly, one electric field travels from electrode element A1 to electrode element P1 when signal generator Y1 is activated, a second electric field travels from electrode element A2 to electrode element P2 when signal generator Y2 is activated, a third electric field travels from electrode element A3 to electrode element P3 when signal generator Y3 is activated, etc. As a result, nine distinct electric fields can travel from respective electrode elements A1-A9 to respective electrode elements P1-P9, depending on which ones of the signal generators Y1-Y9 are activated. When any given single one of the signal generators Y1-Y9 is activated, the resulting electric field will have a comparatively small cross-section and can therefore be analogized to a spotlight.

As described above in connection with the first signal generators X1-X9, more than one of the second signal generators Y1-Y9 can be activated simultaneously (e.g., any 2 at a time, any 3 at a time, etc., up to all 9 at a time). When all nine of the signal generators Y1-Y9 are activated simultaneously, the resulting electric field will have a cross-section that is similar to the electric field that was generated using the prior art transducer arrays 90A/90P. The latter situation can again be analogized to the situation where the light from nine individual spotlights collectively illuminate the same area as a floodlight.

Breaking the electric field up into individual narrower components as described above can be very useful for concentrating the electric field onto the target region. This can be analogized to the way that nine individually controllable spotlights can concentrate illumination onto a given target region more effectively than a single floodlight.

Above and beyond the added level of control that is provided by breaking the electric field up into individual narrower components in order to concentrate the electric field onto the target region, a further level of control over the electric field in the target region can be obtained by controlling the phases of the signals that are generated by the signal generators X1-X9 and Y1-Y9.

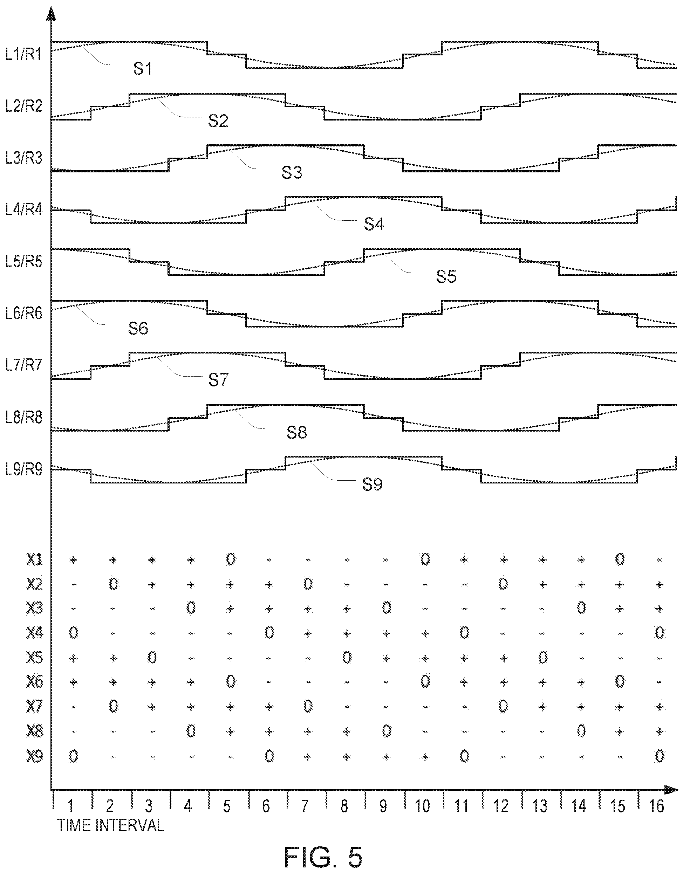

FIG. 5 depicts one example of how the controller 30 can control the phases of the signals that are generated by the signal generators X1-X9 to cause those signal generators to generate output signals that resemble a piecewise approximation to phase-shifted sinusoids S1-S9. More specifically, the controller 30 is programmed to generate control signals in a sequence that causes each of the signal generators X1-X9 to generate an output with a given waveform, so that the output of each of the signal generators X1-X9 is shifted in time with respect to the output of at least one other signal generator X1-X9. An example of the control signals that the controller 30 sends to the signal generator X1 are shown in the bottom half of FIG. 5, with a + representing a command to generate a positive signal, a zero representing a command to generate a zero output, and a - representing a command to generate a negative signal. And the outputs that the signal generators X1-X9 send to the respective sets of electrode elements L1/R1-L9/R9 are shown in the top half of FIG. 5.

When the signal generator X1 receives the +/-/0 control signals depicted in the top row of the bottom half of FIG. 5, the signal generator X1 will generate the digital signal depicted in the top row of the top half of FIG. 5. More specifically, signal generator X1 will (a) apply a positive signal to electrode elements L1 and R1 in time intervals 1-4; (b) apply no signal to those electrode elements in time interval 5; (c) apply a negative signal to those electrode elements in time intervals 6-9; (d) apply no signal to those electrode elements in time interval 10; (e) apply a positive signal to those electrode elements in time intervals 11-14; (e) apply no signal to those electrode elements in time interval 15; and (f) apply a negative signal to those electrode elements in time interval 16. And notably, an overlay of sinusoid S1 with the digital signal depicted in the top row of FIG. 5 makes it clear that this digital signal is a first-order piecewise approximation to sinusoid S1.

The controller 30 sends similar signals to the other signal generators X2-X9, but shifted in time with respect to the signal that is sent to the signal generator X1, as depicted in the bottom half of FIG. 5. The signal generators X2-X9 will respond by applying digital signals to their corresponding electrode element pairs L2/R2 through L9/R9 as depicted in the top half of FIG. 5. And these digital symbols resemble the digital signal described above for the electrode elements L1 and R1, except that they are shifted in time as depicted in the top half of FIG. 5. Here again, an overlay of sinusoids S2-S9 with the digital signals that are applied to the electrode element pairs L2/R2 through L9/R9 make it clear that each of these digital signals is a first-order piecewise approximation of a respective one of the sinusoids S2-S9, and that each of those sinusoids (except S6) is phase-shifted with respect to sinusoid S1.

Optionally, the controller 30 can be programmed to control the shifting in time to steer the electric field within the target region. Under certain conditions, especially when the number of signal generators is large, the ability to generate phase-shifted sinusoids can be used to control the direction of the electric field by implementing beam-steering techniques similar to those used in phased-array radar systems. These techniques can therefore be used to direct the electric field to a target region with an even finer degree of control that can be achieved using the spotlight-shaped electric fields described above in connection with FIG. 3-4.

The operation of the other set of signal generators Y1-Y9 is similar to the operation of the first set of signal generators X1-X9 described above.

Note that while the FIG. 3/4 embodiment is described above in the context of an example that includes nine electrode elements A1-A9, P1-P9, L1-L9, and R1-R9 on each of the transducer arrays 10, the number of electrode elements on each of the transducer arrays 10 can vary (e.g., between 8 and 64). Similarly, instead of the depicted nine first signal generators X1-X9 and nine second signal generators Y1-Y9, the number of first signal generators X can vary, as long as there are at least eight (e.g., between 8 and 64). The number of second signal generators Y can also vary, as long as there are at least eight (e.g., between 8 and 64). The number of signal generators will typically match the number of electrode elements in the corresponding transducer arrays 10.

Finally, in some anatomic locations, only a single pair of transducer arrays 10 is used. In these embodiments, one bank of signal generators (e.g., Y1-Y9 in FIG. 4) and two of the transducer arrays (e.g., 10A and 10P) are omitted.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. An apparatus for applying an electric field to a target region of a subject, comprising:
at least eight first electrode elements (L1-L9);
at least eight second electrode elements (R1-R9);
at least eight electrically-isolated first signal generators (X1-X9), wherein each of the first signal generators (X1-X9) has a respective first control input and is connected to a respective single one of the at least eight first electrode elements (L1-L9) and a respective single one of the at least eight second electrode elements (R1-R9), and each of the first signal generators (X1-X9) is configured, in response to a respective control signal that arrives at the respective first control input, to apply an electrical signal between the respective one of the at least eight first electrode elements (L1-L9) and the respective one of the at least eight second electrode elements (R1-R9); and
a controller (30) that is programmed to generate each of the control signals.

2. The apparatus of claim 1, wherein each of the first signal generators (X1-X9) is configured, in response to a respective first control signal that arrives at the respective first control input, to apply a positive electrical signal between the respective one of the at least eight first electrode elements (L1-L9) and the respective one of the at least eight second electrode elements (R1-R9), and, in response to a respective second control signal that arrives at the respective first control input, to apply a negative electrical signal between the respective one of the at least eight first electrode elements (L1-L9) and the respective one of the at least eight second electrode elements (R1-R9).

3. The apparatus of claim 2, wherein the controller (30) is further programmed, for each of the first signal generators (X1-X9), to apply the respective first control signal and the respective second control signal at respective times in an alternating sequence so that the respective first signal generator (X1-X9) will generate an output that alternates between a positive electrical signal and a negative electrical signal.

4. The apparatus of claim 3, wherein the controller (30) is further programmed to interpose a break in time between each first control signal and each second control signal.

5. The apparatus of claim 1, wherein the controller (30) is further programmed to generate each of the first control signals in a sequence that causes each of the at least eight first signal generators (X1-X9) to generate an output with a given waveform, with the output of each of the at least eight first signal generators (X1-X9) being shifted in time with respect to an output of at least one other of the first signal generators (X1-X9).

6. The apparatus of claim 5, wherein the controller (30) is further programmed to control the shifting in time of the outputs of the first signal generators (X1-X9) to steer an electric field within the target region.

7. The apparatus of claim 5, wherein the controller (30) is further programmed to generate each of the first control signals in a sequence that causes each of the at least eight first signal generators (X1-X9) to generate an output that alternates between a positive electrical signal and a negative electrical signal.

8. The apparatus of claim 1, further comprising:
at least eight third electrode elements (A1-A9);
at least eight fourth electrode elements (P1-P9); and
at least eight electrically-isolated second signal generators (Y1-Y9), wherein each of the second signal generators (Y1-Y9) has a respective second control input and is connected to a respective single one of the at least eight third electrode elements (A1-A9) and a respective single one of the at least eight fourth electrode elements (P1-P9), and each of the second signal generators (Y1-Y9) is configured, in response to a respective control signal that arrives at the respective second control input, to apply an electrical signal between the respective one the of at least eight third electrode elements (A1-A9) and the respective one the of at least eight fourth electrode elements (P1-P9).

9. The apparatus of claim 8, wherein each of the second signal generators (Y1-Y9) is configured, in response to a respective third control signal that arrives at the respective second control input, to apply a positive electrical signal between the respective one of the at least eight third electrode elements (A1-A9) and the respective one of the at least eight fourth electrode elements (P1-P9), and, in response to a respective fourth control signal that arrives at the respective second control input, to apply a negative electrical signal between the respective one of the at least eight third electrode elements (A1-A9) and the respective one of the at least eight fourth electrode elements (P1-P9).

10. The apparatus of claim 9, wherein the controller (30) is further programmed, for each of the second signal generators (Y1-Y9), to apply the respective third control signal and the respective fourth control signal at respective times in an alternating sequence so that the respective second signal generator (Y1-Y9) will generate an output that alternates between a positive electrical signal and a negative electrical signal.

11. The apparatus of claim 10, wherein the controller (30) is further programmed to interpose a break in time between each third control signal and each fourth control signal.

12. The apparatus of claim 9, wherein the controller (30) is further programmed to generate each of the control signals in a sequence that causes each of the at least eight second signal generators (Y1-Y9) to generate an output with a given waveform, with the output of each of the at least eight second signal generators (Y1-Y9) being shifted in time with respect to an output of at least one other of the second signal generators (Y1-Y9).

13. The apparatus of claim 12, wherein the controller (30) is further programmed to control the shifting in time of the outputs of the at least eight second signal generators (Y1-Y9) to steer an electric field within the target region.

14. The apparatus of claim 12, wherein the controller (30) is further programmed to generate each of the control signals in a sequence that causes each of the at least eight second signal generators (Y1-Y9) to generate an output that alternates between a positive electrical signal and a negative electrical signal.

15. The apparatus of claim 8, wherein the controller (30) is further programmed to generate each of the control signals for the at least eight first signal generators (X1-X9) in a sequence that causes each of the at least eight first signal generators (X1-X9) to generate an output with a first given waveform, with the output of each of the at least eight first signal generators (X1-X9) being shifted in time with respect to an output of at least one other of the first signal generators (X1-X9), and each of the control signals for the at least eight second signal generators (Y1-Y9) in a sequence that causes each of the at least eight second signal generators (Y1-Y9) to generate an output with a second given waveform, with the output of each of the at least eight second signal generators (Y1-Y9) being shifted in time with respect to an output of at least one other of the second signal generators (Y1-Y9).

## Patentansprüche

1. Einrichtung zum Anlegen eines elektrischen Feldes an einen Zielbereich einer Zielperson, umfassend:
mindestens acht erste Elektrodenelemente (L1-L9);
mindestens acht zweite Elektrodenelemente (R1-R9);
mindestens acht elektrisch isolierte erste Signalgeneratoren (X1-X9), wobei jeder der ersten Signalgeneratoren (X1-X9) einen jeweiligen ersten Steuereingang aufweist und mit einem jeweiligen einzelnen der mindestens acht ersten Elektrodenelemente (L1-L9) und einem jeweiligen einzelnen der mindestens acht zweiten Elektrodenelemente (R1-R9) verbunden ist, und jeder der ersten Signalgeneratoren (X1-X9) konfiguriert ist, um als Reaktion auf ein jeweiliges Steuersignal, das an dem jeweiligen ersten Steuereingang ankommt, ein elektrisches Signal zwischen dem jeweiligen einen der mindestens acht ersten Elektrodenelemente (L1-L9) und dem jeweiligen einen der mindestens acht zweiten Elektrodenelemente (R1-R9) anzulegen; und
eine Steuereinheit (30), die programmiert ist, um jedes der Steuersignale zu erzeugen.

2. Einrichtung nach Anspruch 1, wobei jeder der ersten Signalgeneratoren (X1-X9) konfiguriert ist, um als Reaktion auf ein jeweiliges erstes Steuersignal, das an dem jeweiligen ersten Steuereingang ankommt, ein positives elektrisches Signal zwischen dem jeweiligen einen der mindestens acht ersten Elektrodenelemente (L1-L9) und dem jeweiligen einen der mindestens acht zweiten Elektrodenelemente (R1-R9) anzulegen und als Reaktion auf ein jeweiliges zweites Steuersignal, das an dem jeweiligen ersten Steuereingang ankommt, ein negatives elektrisches Signal zwischen dem jeweiligen einen der mindestens acht ersten Elektrodenelemente (L1-L9) und dem jeweiligen einen der mindestens acht zweiten Elektrodenelemente (R1-R9) anzulegen.

3. Einrichtung nach Anspruch 2, wobei die Steuereinheit (30) weiter programmiert ist, um für jeden der ersten Signalgeneratoren (X1-X9) das jeweilige erste Steuersignal und das jeweilige zweite Steuersignal zu jeweiligen Zeitpunkten in einer abwechselnder Reihenfolge anzulegen, sodass der jeweilige erste Signalgenerator (X1-X9) eine Ausgabe erzeugt, die zwischen einem positiven elektrischen Signal und einem negativen elektrischen Signal wechselt.

4. Einrichtung nach Anspruch 3, wobei die Steuereinheit (30) weiter programmiert ist, um zwischen jedem ersten Steuersignal und jedem zweiten Steuersignal eine Zeitpause einzufügen.

5. Einrichtung nach Anspruch 1, wobei die Steuereinheit (30) weiter programmiert ist, um jedes der ersten Steuersignale in einer Reihenfolge zu erzeugen, die bewirkt, dass jeder der mindestens acht ersten Signalgeneratoren (X1-X9) eine Ausgabe mit einer vorgegebenen Wellenform erzeugt, wobei die Ausgabe jedes der mindestens acht ersten Signalgeneratoren (X1-X9) in Bezug auf eine Ausgabe mindestens eines anderen der ersten Signalgeneratoren (X1-X9) zeitlich versetzt ist.

6. Einrichtung nach Anspruch 5, wobei die Steuereinheit (30) weiter programmiert ist, um das zeitliche Versetzen der Ausgaben der ersten Signalgeneratoren (X1-X9) zu steuern, um ein elektrisches Feld innerhalb des Zielbereichs zu lenken.

7. Einrichtung nach Anspruch 5, wobei die Steuereinheit (30) weiter programmiert ist, um jedes der ersten Steuersignale in einer Reihenfolge zu erzeugen, die bewirkt, dass jeder der mindestens acht ersten Signalgeneratoren (X1-X9) eine Ausgabe erzeugt, die zwischen einem positiven elektrischen Signal und einem negativen elektrischen Signal wechselt.

8. Einrichtung nach Anspruch 1, weiter umfassend:
mindestens acht dritte Elektrodenelemente (A1-A9);
mindestens acht vierte Elektrodenelemente (P1-P9); und
mindestens acht elektrisch isolierte zweite Signalgeneratoren (Y1-Y9), wobei jeder der zweiten Signalgeneratoren (Y1-Y9) einen jeweiligen zweiten Steuereingang aufweist und mit einem jeweiligen einzelnen der mindestens acht dritten Elektrodenelemente (A1-A9) und einem jeweiligen einzelnen der mindestens acht vierten Elektrodenelemente (P1-P9) verbunden ist, und jeder der zweiten Signalgeneratoren (Y1-Y9) konfiguriert ist, um als Reaktion auf ein jeweiliges Steuersignal, das an dem jeweiligen zweiten Steuereingang ankommt, ein elektrisches Signal zwischen dem jeweiligen einen der mindestens acht dritten Elektrodenelemente (A1-A9) und dem jeweiligen einen der mindestens acht vierten Elektrodenelemente (P1-P9) anzulegen.

9. Einrichtung nach Anspruch 8, wobei jeder der zweiten Signalgeneratoren (Y1-Y9) konfiguriert ist, um als Reaktion auf ein jeweiliges drittes Steuersignal, das an dem jeweiligen zweiten Steuereingang ankommt, ein positives elektrisches Signal zwischen dem jeweiligen einen der mindestens acht dritten Elektrodenelemente (A1-A9) und dem jeweiligen einen der mindestens acht vierten Elektrodenelemente (P1-P9) anzulegen und als Reaktion auf ein jeweiliges viertes Steuersignal, das an dem jeweiligen zweiten Steuereingang ankommt, ein negatives elektrisches Signal zwischen dem jeweiligen einen der mindestens acht dritten Elektrodenelemente (A1-A9) und dem jeweiligen einen der mindestens acht vierten Elektrodenelemente (P1-P9) anzulegen.

10. Einrichtung nach Anspruch 9, wobei die Steuereinheit (30) weiter programmiert ist, um für jeden der zweiten Signalgeneratoren (Y1-Y9) das jeweilige dritte Steuersignal und das jeweilige vierte Steuersignal zu jeweiligen Zeitpunkten in einer abwechselnder Reihenfolge anzulegen, sodass der jeweilige zweite Signalgenerator (Y1-Y9) eine Ausgabe erzeugt, die zwischen einem positiven elektrischen Signal und einem negativen elektrischen Signal wechselt.

11. Einrichtung nach Anspruch 10, wobei die Steuereinheit (30) weiter programmiert ist, um zwischen jedem dritten Steuersignal und jedem vierten Steuersignal eine Zeitpause einzufügen.

12. Einrichtung nach Anspruch 9, wobei die Steuereinheit (30) weiter programmiert ist, um jedes der Steuersignale in einer Reihenfolge zu erzeugen, die bewirkt, dass jeder der mindestens acht zweiten Signalgeneratoren (Y1-Y9) eine Ausgabe mit einer vorgegebenen Wellenform erzeugt, wobei die Ausgabe jedes der mindestens acht zweiten Signalgeneratoren (Y1-Y9) in Bezug auf die Ausgabe mindestens eines anderen der zweiten Signalgeneratoren (Y1-Y9) zeitlich versetzt ist.

13. Einrichtung nach Anspruch 12, wobei die Steuereinheit (30) weiter programmiert ist, um das zeitliche Versetzen der Ausgaben der mindestens acht zweiten Signalgeneratoren (Y1-Y9) zu steuern, um ein elektrisches Feld innerhalb des Zielbereichs zu lenken.

14. Einrichtung nach Anspruch 12, wobei die Steuereinheit (30) weiter programmiert ist, um jedes der Steuersignale in einer Reihenfolge zu erzeugen, die bewirkt, dass jeder der mindestens acht zweiten Signalgeneratoren (Y1-Y9) eine Ausgabe erzeugt, die zwischen einem positiven elektrischen Signal und einem negativen elektrischen Signal wechselt.

15. Einrichtung nach Anspruch 8, wobei die Steuereinheit (30) weiter programmiert ist, um jedes der Steuersignale für die mindestens acht ersten Signalgeneratoren (X1-X9) in einer Reihenfolge, die bewirkt, dass jeder der mindestens acht ersten Signalgeneratoren (X1-X9) eine Ausgabe mit einer ersten vorgegebenen Wellenform erzeugt, wobei die Ausgabe jedes der mindestens acht ersten Signalgeneratoren (X1-X9) in Bezug auf die Ausgabe mindestens eines anderen der ersten Signalgeneratoren (X1-X9) zeitlich versetzt ist, und jedes der Steuersignale für die mindestens acht zweiten Signalgeneratoren (Y1-Y9) in einer Reihenfolge zu erzeugen, die bewirkt, dass jeder der mindestens acht zweiten Signalgeneratoren (Y1-Y9) eine Ausgabe mit einer zweiten gegebenen Wellenform erzeugt, wobei die Ausgabe jedes der mindestens acht zweiten Signalgeneratoren (Y1-Y9) in Bezug auf die Ausgabe mindestens eines anderen der zweiten Signalgeneratoren (Y1-Y9) zeitlich versetzt ist.

## Revendications

1. Appareil permettant d'appliquer un champ électrique à une région cible d'un sujet, comprenant :
au moins huit premiers éléments d'électrode (L1-L9) ;
au moins huit deuxièmes éléments d'électrode (R1-R9) ;
au moins huit premiers générateurs de signal électriquement isolés (X1-X9), dans lequel chacun des premiers générateurs de signal (X1-X9) possède une première entrée de commande respective et est connecté à un seul des au moins huit premiers éléments d'électrode (L1-L9) et à un seul des au moins huit deuxièmes éléments d'électrode (R1-R9), et chacun des premiers générateurs de signal (X1-X9) est configuré, en réponse à un signal de commande respectif arrivant à la première entrée de commande respective, pour appliquer un signal électrique entre l'un des au moins huit premiers éléments d'électrode (L1-L9) et l'un des au moins huit deuxièmes éléments d'électrode (R1-R9) respectifs ; et
un dispositif de commande (30) qui est programmé pour générer chacun des signaux de commande.

2. Appareil selon la revendication 1, dans lequel chacun des premiers générateurs de signal (X1-X9) est configuré, en réponse à un premier signal de commande respectif arrivant à la première entrée de commande respective, pour appliquer un signal électrique positif entre l'un des au moins huit premiers éléments d'électrode (L1-L9) et l'un des au moins huit deuxièmes éléments d'électrode (R1-R9), et, en réponse à un deuxième signal de commande respectif arrivant à la première entrée de commande respective, pour appliquer un signal électrique négatif entre l'un des au moins huit premiers éléments d'électrode (L1-L9) et l'un des au moins huit deuxièmes éléments d'électrode (R1-R9) respectifs.

3. Appareil selon la revendication 2, dans lequel le dispositif de commande (30) est en outre programmé, pour chacun des premiers générateurs de signal (X1-X9), pour appliquer le premier signal de commande respectif et le deuxième signal de commande respectif à des moments respectifs dans une séquence alternée de sorte que le premier générateur de signal respectif (X1-X9) génère une sortie qui alterne entre un signal électrique positif et un signal électrique négatif.

4. Appareil selon la revendication 3, dans lequel le dispositif de commande (30) est en outre programmé pour interposer une pause temporelle entre chaque premier signal de commande et chaque deuxième signal de commande.

5. Appareil selon la revendication 1, dans lequel le dispositif de commande (30) est en outre programmé pour générer chacun des premiers signaux de commande dans une séquence qui amène chacun des au moins huit premiers générateurs de signal (X1-X9) à générer une sortie avec une forme d'onde donnée, la sortie de chacun des au moins huit premiers générateurs de signal (X1-X9) étant décalée dans le temps par rapport à la sortie d'au moins un autre des premiers générateurs de signal (X1-X9).

6. Appareil selon la revendication 5, dans lequel le dispositif de commande (30) est en outre programmé pour commander le décalage temporel des sorties des premiers générateurs de signal (X1-X9) afin de diriger un champ électrique dans la région cible.

7. Appareil selon la revendication 5, dans lequel le dispositif de commande (30) est en outre programmé pour générer chacun des premiers signaux de commande dans une séquence qui amène chacun des au moins huit premiers générateurs de signal (X1-X9) à générer une sortie qui alterne entre un signal électrique positif et un signal électrique négatif.

8. Appareil selon la revendication 1, comprenant en outre :
au moins huit troisièmes éléments d'électrode (A1-A9) ;
au moins huit quatrièmes éléments d'électrode (P1-P9) ; et
au moins huit deuxièmes générateurs de signal électriquement isolés (Y1-Y9), dans lequel chacun des deuxièmes générateurs de signal (Y1-Y9) présente une entrée de commande respective et est connecté à un seul des au moins huit troisièmes éléments d'électrode (A1-A9) et à un seul des au moins huit quatrièmes éléments d'électrode (P1-P9), et chacun des deuxièmes générateurs de signal (Y1-Y9) est configuré, en réponse à un signal de commande respectif arrivant à l'entrée de commande respective, pour appliquer un signal électrique entre l'un des au moins huit troisièmes éléments d'électrode (A1-A9) et l'un des au moins huit quatrièmes éléments d'électrode (P1-P9) respectifs.

9. Appareil selon la revendication 8, dans lequel chacun des deuxièmes générateurs de signal (Y1-Y9) est configuré, en réponse à un troisième signal de commande respectif qui arrive à la deuxième entrée de commande respective, pour appliquer un signal électrique positif entre l'un des au moins huit troisièmes éléments d'électrode (A1-A9) et l'un des au moins huit quatrièmes éléments d'électrode (P1-P9), et, en réponse à un quatrième signal de commande respectif qui arrive à la deuxième entrée de commande respective, pour appliquer un signal électrique négatif entre l'un des au moins huit troisièmes éléments d'électrode (A1-A9) et l'un des au moins huit quatrièmes éléments d'électrode (P1-P9) respectifs.

10. Appareil selon la revendication 9, dans lequel le dispositif de commande (30) est en outre programmé, pour chacun des deuxièmes générateurs de signal (Y1-Y9), pour appliquer le troisième signal de commande respectif et le quatrième signal de commande respectif à des moments respectifs dans une séquence alternée de sorte que le deuxième générateur de signal respectif (Y1-Y9) génère une sortie qui alterne entre un signal électrique positif et un signal électrique négatif.

11. Appareil selon la revendication 10, dans lequel le dispositif de commande (30) est en outre programmé pour interposer une pause temporelle entre chaque troisième signal de commande et chaque quatrième signal de commande.

12. Appareil selon la revendication 9, dans lequel le dispositif de commande (30) est en outre programmé pour générer chacun des signaux de commande dans une séquence qui amène chacun des au moins huit deuxièmes générateurs de signal (Y1-Y9) à générer une sortie avec une forme d'onde donnée, la sortie de chacun des au moins huit deuxièmes générateurs de signal (Y1-Y9) étant décalée dans le temps par rapport à la sortie d'au moins un autre des deuxièmes générateurs de signal (Y1-Y9).

13. Appareil selon la revendication 12, dans lequel le dispositif de commande (30) est en outre programmé pour commander le décalage temporel des sorties des au moins huit deuxièmes générateurs de signal (Y1-Y9) afin de diriger un champ électrique dans la région cible.

14. Appareil selon la revendication 12, dans lequel le dispositif de commande (30) est en outre programmé pour générer chacun des signaux de commande dans une séquence qui amène chacun des au moins huit deuxièmes générateurs de signal (Y1-Y9) à générer une sortie qui alterne entre un signal électrique positif et un signal électrique négatif.

15. Appareil selon la revendication 8, dans lequel le dispositif de commande (30) est en outre programmé pour générer chacun des signaux de commande des au moins huit premiers générateurs de signal (X1-X9) dans une séquence qui amène chacun des au moins huit premiers générateurs de signal (X1-X9) à générer une sortie avec une première forme d'onde donnée, la sortie de chacun des au moins huit premiers générateurs de signal (X1-X9) étant décalée dans le temps par rapport à la sortie d'au moins un autre des premiers générateurs de signal (X1-X9), et chacun des signaux de commande des au moins huit deuxièmes générateurs de signal (Y1-Y9) dans une séquence qui amène chacun des au moins huit deuxièmes générateurs de signal (Y1-Y9) à générer une sortie avec une deuxième forme d'onde donnée, la sortie de chacun des au moins huit deuxièmes générateurs de signal (Y1-Y9) étant décalée dans le temps par rapport à la sortie d'au moins un autre des deuxièmes générateurs de signal (Y1-Y9).
